(19) Europäisches Patentamt
European Patent Office
Office européen des brevets

(11) Publication number: 0 297 924
A2

(12) **EUROPEAN PATENT APPLICATION**

(21) Application number: 88306055.0

(22) Date of filing: 01.07.88

(51) Int. Cl.4: C 12 P 21/02
C 12 N 15/00, A 61 K 39/42,
A 61 K 39/285

(30) Priority: 01.07.87 US 68533

(43) Date of publication of application:
04.01.89 Bulletin 89/01

(84) Designated Contracting States:
AT BE CH DE ES FR GB GR IT LI LU NL SE

(71) Applicant: CITY OF HOPE
1500 East Duarte Road
Duarte California 91010-0269 (US)

(72) Inventor: Cantin, Edouard Maxime
1160 Colorado Boulevard
Los Angeles, California 90041 (US)

(74) Representative: Mercer, Christopher Paul et al
Carpmaels & Ransford 43, Bloomsbury Square
London WC1A 2RA (GB)

(54) **Recombinant vaccinia virus expressing HSV 1 gB.**

(57) The HSV 1 (F) gB gene was isolated and modified at the 5' end by in vitro oligonucleotide directed mutagenesis. The modified gB gene was inserted into the vaccinia virus genome and expressed under the control of a vaccinia virus promoter. The mature gB glycoprotein produced by the vaccinia virus recombinant was glycosylated, expressed at the cell surface and was indistinguishable from authentic HSV 1 gB. Mice immunized intradermally and ocularly with the recombinant vaccinia virus produced gB specific neutralizing antibodies and were resistant to a lethal and latent HSV 1 challenge.

FIG.1

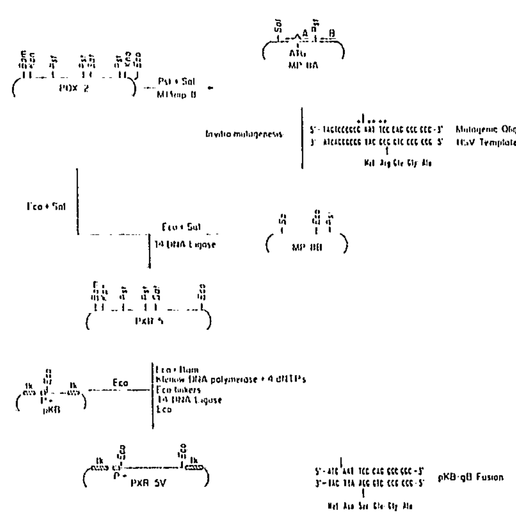


EP 0 297 924 A2

## Description

# RECOMBINANT VACCINIA VIRUS EXPRESSING HSV 1 gB

This invention relates to recombinant vaccinia virus which expresses authentic HSV 1 gB and induces a protective immune response.

## Background of the Invention

The herpes simplex virus type 1 (HSV 1) genome codes for four antigenically distinct glycoproteins: gB, gC, gD and gE, all of which appear to have homologues in HSV 2. Although the DNA sequence of all four glycoprotein genes has been determined and structural information has been obtained, relatively little is known about their function.

The purified gB glycoprotein of HSV 1, a major antigenic determinant, has been shown to elicit protective immunity to HSV 1 infection in mice. In addition, passive immunization of mice with gB specific monoclonal antibodies having HSV 1 neutralizing activity has been shown to be protective against infection with both HSV 1 and HSV 2. This information indicates that gB is an important inducer of protective immunity to HSV infection and that it plays an essential role in the process of HSV penetration into cells.

Recently, recombinant vaccinia virus expressing gD has been shown to immunize mice effectively against lethal and latent HSV infection. This invention provides recombinant vaccinia virus which expresses authentic HSV 1 gB on the surface of infected cells, presents this antigen to the immune system in a manner analogous to that in a normal HSV 1 infection, and produces an immune response in mice effective against lethal and latent HSV 1 infection. Administration of an effective amount of the recombinant vaccinia virus of this infection thus provides a means for immunizing mammals, including man, against HSV 1 infection.

## Summary of the Invention

The HSV 1 (F) gB gene was isolated and modified at the 5′ end by in vitro oligonucleotide directed mutagenesis. The modified gB gene was inserted into the vaccinia virus genome and expressed under the control of a vaccinia virus promoter. The mature gB glycoprotein produced by the vaccinia virus recombinant was glycosylated, expressed at the cell surface and was indistinguishable from authentic HSV 1 gB . Mice immunized intradermally and ocularly with the recombinant vaccinia virus produced gB specific neutralizing antibodies and were resistant to a lethal and latent HSV 1 challenge.

## Description of the Figures

Figure 1-Construction of a recombinant vaccinia virus vector for insertion of the HSV 1 gB coding sequence into vaccinia virus.

The HSV 1 gB coding sequence is contained in the 3-4kb XhoI Bam HI fragment in the plasmid POX 2. Only those restriction sites relevant to various plasmid constructs are shown (not to scale). The sequence of the mutagenic oligonucleotide (A, 25 mer) and that of the corresponding target region in the gB gene are shown. Mismatches corresponding to the new EcoRI site are indicated with filled circles. In vitro oligonucleotide directed mutagenesis was carried out by the double primer method, using M13 universal sequencing primer (B). Mutant phages were identified by hybridization with the $^{32}P$ labelled mutagenic oligonucleotide (A) and verified by restriction analysis (MP-8B). The in frame fusion resulting from insertion of the modified gB gene into the vaccinia virus vector pKB is shown on the last line together with the amino acid sequence of the mutant gB polypeptide.

Figure 2-Characterization of HSV 1 gB glycoprotein synthesized by the recombinant vaccinia virus V11 (gB).

(A) Immunoblot Analysis. Vero cell monolayers were infected with the vaccinia influenza recombinant (V36) or with the vaccinia gB recombinant (V11) or with HSV 1 (F). After 16 hours, cell extracts were prepared from infected and uninfected cells and resolved on a 10% polyacrylamide gel. The separated polypeptides were electrophoretically transferred to nitrocellulose strips and then reacted sequentially with either anti-HSV 1 serum, anti-gB serum or normal rabbit serum, and then with $^{125}I$-SPA. Reactive antigens were visualized by autoradiography. The lane labelled HSV shows 14 C-glucosamine labelled HSV 1 glycoproteins resolved in the same gel.

(B) Immunoprecipitation. Vero cells infected with V11, V36 or HSV 1 were metabolically labelled with 14 C-glucosamine for 20 hours. Cytoplasmic antigens (lane 1) were immunoprecipitated with rabbit anti-HSV 1 serum (lane 2) or rabbit anti-gB serum (lane 3), or normal rabbit serum (lane 4), and the immune precipitates were resolved on 10% polyacrylamide gels. An autoradiogram is shown, and the mobility of the gB, gC and gD HSV 1 glycoproteins are indicated.

Figure 3-Cell surface immunofluorescent staining of gB.

Cover slip cultures of infected and uninfected CV-1 cells as described hereinafter.

## Detailed Description of the Invention

### Plasmid constructions and oligonucleotide directed mutagenesis

Construction of recombinant plasmids and transformation of Escherichia coli were essentially as described by Maniatis.[1] The gB gene was isolated from the Bam HI G fragment (.345-.399 map units) which was subcloned from the HSV 1 (F) Bgl II (I) fragment cloned in phage charon 28.

Alteration of the HSV 1 gB gene and its insertion into the vaccinia vector pKB was achieved as outlined in Figure 1. The gB translational initiation codon (ATG) was converted into an EcoRI site, such that upon ligation to EcoRI cleaved pKB,[1] an in frame fusion to a vaccinia derived translational initiation codon (ATG) would result. A 3.4 kb Bam HI/Xho I fragment (.345 - .368 map units) containing the entire HSV 1 (F) gB gene was subcloned from the Bam HI G fragment to give the plasmid POX 2. A 1.2 kb Sal I/Pst I fragment (.360 - .368 map units) comprising sequences encoding the amino terminal region of gB, was subcloned into M13 mp-8 to give mp-8A. The insert was sequenced from the Pst I site to just past the gB ATG initiation codon, using the dideoxy technique. The sequence obtained was completely homologous to the published sequence for HSV 1 (F) gB, between nucleotide residues 562 through 838.[1] The DNA sequence was used to design a mutagenic oligonucleotide (25 mer) for conversion of the gB ATG to an EcoRI site (Figure 1). In vitro oligonucleotide mutagenesis by the double primer method[2] was carried out using single stranded mp-8A phage DNA as a template and the mutation was verified by restriction enzyme analysis. This alteration (Figure 1) converted the second amino acid residue from argenine to serine and insertion of a new amino acid, asparagine, such that the gB polypeptide was one amino acid longer.

To reconstruct the HSV 1 gB gene (Figure 1), the 960 bp EcoRI/Sal I fragment from mp-8B was ligated to the large EcoRI/Sal I fragment isolated from POX-2, and following transformation of E.coli, the plasmid PXR-5 was obtained. The 3.2 kb EcoRI/BamHI insert fragment from PXR-5 was isolated and cloned into the EcoRI site of the vaccinia plasmid vector pKB to give PXR-5V. The vaccinia pKB vector comprises the 11k late vaccinia promotor and transcription start site immediately downstream of which is an EcoRI site into which heterologous genes can be inserted (Chakrabarti, et al., supra). This vaccinia transcriptional unit is flanked by vaccinia virus TK gene sequences which facilitate homologous recombination into the TK gene locus of the vaccinia virus genome. Insertion of the vaccinia HSV 1 gB transcriptional unit into the vaccinia virus genome was achieved by transfecting vaccinia virus infected CV-1 cells within the plasmid PCR-5V, and then selecting TK-recombinant vaccinia virus progeny on TK- cells in the presence of BrdUrd. True recombinant TK- vaccinia virus was distinguished from spontaneous TK- vaccinia mutants by screening for the presence of HSV 1 gB DNA sequences. A recombinant vaccinia virus which expressed the HSV 1 gB gene was isolated and plaque purified thrice in TK- cells in the presence of 5-bromo-2-deoxyuridine (BrdUrd). A single plaque was then used to prepare, under non-selective conditions, a large stock of the recombinant vaccinia gB virus which was designated V11.

In the altered gB polypeptide, the gB coding sequences were fused in frame to a vaccinia virus derived translational initiation codon (ATG), thus providing efficient expression of the HSV 1 gB gene using vaccinia virus late transcriptional regulatory sequences. The altered amino acids are in the portion of the gB precursor polypeptide which is thought to contain a signal sequence directing the transport of gB to the cell surface.[3] Although this portion is cleaved off during maturation, alteration in the putative signal sequence apparently did not affect the maturation of gB and the localization of gB to the cell membrane. Evidence that gB maturation and localization were normal includes data showing that (1) gB produced by the recombinant is antigenic and glycosylated, and co-migrates in SDS-PAGE with authentic HSV 1 (F) gB (Figures 2A and 2B), (2) the gB glycoprotein is expressed on the cell surface membrane following infection of cells with the recombinant vaccinia virus V11 (Figure 3), and (3) immunization of mice with V11 results in production of serum antibodies which bind specifically to purified HSV 1 gB in an ELISA assay and which are capable of neutralizing HSV 1 infectivity (Tables I and II). Moreover, immunization with V11 protected mice against a lethal HSV 1 challenge with an efficacy equivalent to that of the vaccinia gD recombinant virus. Taken together, these results indicate that cleavage of the leader peptide occurred and that the mature gB glycoprotein expressed by V11 is identical to that of authentic gB expressed by HSV 1.

[1] See, e.g., Maniatis, T., et al. (1982). Pages 86-96. Cold Spring Harbor Laboratory. See Chakrabarti, S., et al., Mol.Cell Biol. 5:3403-3409 (1985). See Bzik, D.J., et al., Virology 133:301-314 (1984).

[2] See, e.g., Messing, J., In: Methods Enzymology 101:20-78 (1983) and Smith, M., In: Ann. Rev. Genetics 19:423-462 Annual Reviews, Inc. (1985).

[3] See Frank, R.J., et al., J.Virol. 45:634 (1983); Bzik, D.J., et al., Virology 133:301-314 (1984); Perlman, D., et al., J.Mol. Biol. 167:391-409 (1983); and von Heijne, G., J.Mol.Biol. 173:243-251 (1984).

Immunoblotting, radioimmunoprecipitation and protein analysis

Infected and uninfected Vero cell extracts were prepared for immunoblot analysis using procedures previously described in detail.[4] Infected cell polypeptides electrophoretically separated in SDS-polyacrylamide gels (SDS-PAGE) were electrophoretically blotted onto nitrocellulose membranes at 500 mAmp for 1.5 hours. Following transfer, the nitrocellulose sheets were blocked with BSA, reacted with antiserum and bound antibody detected with $^{125}$I-staphlococcal protein A ($^{125}$I SPA) (Eberle, supra (1983)). After drying the nitrocellulose membrane, bound $^{125}$I-SPA was detected by autoradiography with Kodak X-Omat AR-2 film.

Antigens used in radioimmunoprecipitation assays were prepared from infected Vero cell monolayers metabolically labelled with $^{14}$C-glucosamine (2.5 Ci/ml) from 4-24 hours post-infection as previously described.[5] Antigens were preadsorbed with SPA (Pansorbin, Calbiochem, La Jolla, CA) for one hour at room temperature. SPA was then removed by centrifugation at 12,000 X g for 5 minutes and the antigen preparation reacted with rabbit antiserum for one hour at 37° C and 6 hours at 4° C. Immune complexes were precipitated by the addition of SPA, and after washing extensively, the immune precipitates were analyzed by SDS-PAGE (Eberle, supra (1981)).

All SDS-polyacrylamide gels were either 10% acrylamide cross-linked with DATD or 8% acrylamide cross-linked with BIS (Eberle, supra (1980)).

The pgB and gB glycoproteins comprise the same polypeptide chain but differ only in the extent of glycosylation.[6] To characterize the gB protein expressed by the vaccinia recombinant, Vero cells were infected with vaccinia virus recombinant V11 or V36, which express the HSV 1 gB glycoprotein and the influenza hemagglutinin (HA) glycoprotein respectively, or with HSV 1. Cell extracts prepared from infected and uninfected Vero cells were resolved by SDS-PAGE, and the separated proteins transferred to nitrocellulose membranes. Nitrocellulose strips, each containing identical sets of proteins, were reacted first with rabbit anti-HSV serum, rabbit anti-gB serum or normal rabbit serum, and then with $^{125}$I-SPA. In the autoradiogram shown in Figure 2A, a major band corresponding to the pgB glycoprotein and a minor band corresponding to the gB glycoprotein were visible only in V11 or HSV 1 infected cells and were detected only with monospecific anti-gB serum or anti-HSV serum. HSV 1 infected cell extract reacted with anti-HSV serum detected numerous additional HSV 1 antigens; whereas, V36 infected cells and uninfected cells did not react with any of the antisera used. Figure 2A also demonstrates that the pgB and gB glycoproteins produced by the vaccinia recombinant V11 have electrophoretic mobilities which are indistinguishable from those of authentic HSV 1 pgB and gB.

To ascertain whether gB produced by the recombinant vaccinia virus V11 was glycosylated, cells infected with V11, V36 and HSV 1, as well as uninfected cells, were labelled with $^{14}$C-glucosamine. Detergent soluble antigens from these cells were immunoprecipitated with rabbit anti-HSV serum, rabbit anti-gB serum, or normal rabbit serum. SDS-PAGE analysis of the immunoprecipitated proteins demonstrated that the gB glycoprotein produced by the vaccinia recombinant V11 were both glycosylated and had an electrophoretic mobility identical to authentic HSV 1 gB (Figure 2B). No proteins from V36 infected or uninfected cells were bound by the antisera used, even though V36 does produce a major glycosylated species (Figure 2B).

Membrane immunofluorescence

Cover slip cultures of CV-1 cells in 60 mm plates were seeded 36 hours before infection so as to be approaching confluency at the time of straining. Monolayers were infected with an input multiplicity of 0.1 plaque forming unit (PFU) per cell, and the virus was allowed to adsorb for one hour at 37° C, after which the cells were washed with phosphate buffered saline (PBS) and maintenance medium added. At 18 hours post-infection, cover slips were washed three times with PBS at room temperature, excess PBS was removed, and 15 ul of diluted rabbit antiserum was added. After 30 minutes at room temperature in a humidified chamber, cover slips were washed three times in PBS, and the procedure was repeated with FITC-goat anti-rabbit IgG. After the final washings, cover slips were mounted on slides with PBS-glycerol (4:1). Fluorescence was observed within two hours on a Zeiss microscope. Photographs were taken with Kodak Ektachrome daylight ASA 1000 film. See Figure 3.

In a productive HSV 1 infection, the gB glycoprotein is incorporated into the various membrane systems of the cell, including the plasma membrane where gB is expressed on the external surface of the cell. Immunofluorescence studies were undertaken to determine whether gB specified by the vaccinia recombinant V11 was similarly expressed on the plasma membrane of infected cells. Cover slip cultures of CV 1 cells uninfected and infected with V11 (gB), V52 (gD) or HSV 1 were reacted with rabbit anti-gB serum followed by fluorescein-conjugated goat anti-rabbit IgG. As shown in Figure 3, strong membrane immunofluorescence was seen only with V11 (Figure 3A) and HSV (Figure 3C) infected cells reacted with anti-gB serum. Cells infected with V36 (Influenza HA) and reacted with anti-gB serum, or V11 infected cells reacted with normal rabbit serum were negative.

[4] See, e.g., Eberle, R., et al., J.Virol. 35:902-917 (1980) and Eberle, R., et al., J.Inf.Dis. 148:436-444 (1983).
[5] See, e.g., Eberle, R., et al., Infec.Immun. 31:1062-1070 (1981).
[6] See Spear, P.G., In: The Herpesviruses (B. Roizmann, ed.) 3:315-356, Plenum, New York (1984).

ELISA assay

Immulon II microtiter plates (Dynatech Laboratories, Alexandria, VA) were coated with purified HSV 1 gB diluted in carbonate buffer. Immediately prior to use, coated wells were blocked with 2% BSA in PBS for 1 hour at 37°C. Mouse sera were diluted in the same buffer and 50 ul added to each well. Following a two-hour incubation at 37°C, plates were washed with 0.05% Tween 20 in PBS, and bound murine IgG was detected with a commercial avidin-biotin kit (Vector Laboratories, Burlingame, CA). Optical density (OD 490) values greater than twice the value obtained with serum from non-immunized mice was considered significant.

Immunization of animals and HSV 1 challenge

Male BALB/c mice (Jackson Labs) 6-8 weeks old were immunized with 3 X $10^7$ plaque forming units (PFU) of either vaccinia HSV 1 gB (V11), vaccinia HSV 1 gD (V52) or vaccinia influenza hemagglutinin (V36). Immunizations were by the intradermal route at the base of the tail. Sera from immunized mice were collected at four weeks post-immunization and pooled for each immunization group. HSV 1 neutralizing antibody titers were determined by a complement-independent plaque reduction assay. Mouse serum was diluted two-fold in 500 ul of media and then 100 PFU of HSV 1 was incubated with each serum dilution for 1 hour at 37°C. A total of 400 ul was then added to monolayers of Vero cells and plaqued using methylcellulose. Endpoints were expressed as the reciprocal of the highest serum two-fold dilution reducing the number of plaques by 50%. At 6 weeks after immunization, mice were challenged by the intraperitoneal injection of 1 X $10^4$ PFU of HSV 1 (McKrae) and observed for indication of mortality for 14 days.

An ELISA assay was used to measure the level of serum antibodies capable of specific binding to purified HSV 1 gB. BALB/c mice were immunized by the intradermal route with 3 X $10^7$ PFU of each of the vaccinia virus recombinants.V11 (gB), V52 (gD) or V36 (Influenza HA). Sera was collected and pooled four weeks after viral inoculation and assayed for anti-gB antibodies. The assay data is reported in Table I.

Table I

Immunization with Recombinant Vaccinia Viruses: Serum Antibody Titers and Resistance to Lethal HSV 1 Challenge

| | SERUM ANTIBODY TITER** | | HSV 1 CHALLENGE*** | |
|---|---|---|---|---|
| IMMUNIZATION* | NEUTRALIZATION | ANTI-gB | NUMBER OF MICE | PERCENT MORTALITY |
| Non-Immunized | 4 | <10± | 10 | 100 |
| Vaccinia V52 (gD) | 256 | <10 | 20 | 5 |
| Vaccinia V11 (gB) | 32 | ≥160 | 17 | 6 |
| Vaccinia V36 (influenza HA) | ND | ND | 10 | 70 |

*Balb/c mice were immunized by the intradermal injection at the base of the tail with 3 X $10^7$ PFU of the vaccinia virus recombinants.

Table I shows that only mice immunized with the V11 recombinant or HSV 1 developed serum antibodies to gB. Neutralizing antibody titers were lower for V11 (gB) immunized mice as compared to V52 (gD) immunized mice. Mice were then challenged by intraperitoneal injection of HSV 1. There was 90% survival in mice immunized with either V11 (gB) or V52 (gD), while a high degree of mortality occurred in non-immunized mice and in mice immunized with V36.

The effect of vaccinia recombinant virus vaccination on the acute and latent ganglionic infection in mice following ocular challenge with HSV 1 is reported in Table II.

Table II

| Vaccination Group | Number Mice | Percent Mortality | Percent Acute Infection (a) | Percent Latent Infection (b) |
|---|---|---|---|---|
| VII (gB) | 48 | 0 | 15 | 41 |
| V52 (gD) | 48 | 0 | 10 | 5 |
| V36 (influenza HA) | 40 | 88 | 95 | 100 |
| Non-Vaccinated | 20 | 100 | 100 | 75(c) |

(a) Acute ganglionic infection determined by isolation of HSV from the supernatant fluid of disrupted trigeminal ganglia from mice at 5 days after ocular challenge with HSV 1.

(b) Latent ganglionic infection determined by the isolation of HSV from minced trigeminal ganglia at 30 days after ocular challenge with HSV 1.

(c) Data collected from mice infected by the ocular route with 1 $\log_{10}$ dose of HSV 1 strain McKrae than used in the other vaccination groups.

The invention accordingly subsumes a method for immunizing mammals, including man, against HSV 1 by administration, preferably intradermally, of an effective amount of the recombinant vaccinia virus of this invention.

**Claims**

1. A recombinant vaccinia virus which expresses gB glycoprotein substantially identical to that expressed by HSV 1.
2. gB glycoprotein which is expressed by a recombinant vaccinia virus and which is substantially identical to that expressed by HSV 1.
3. The recombinant vaccinia virus of claim 1, for use in immunising a mammal against HSV 1 infection.
4. The gB glycoprotein of claim 2, for use in immunising a mammal against HSV 1 infection.
5. The virus of claim 3 or the gB glycoprotein of claim 4, for use in immunising a human.

0297924

FIG. 1

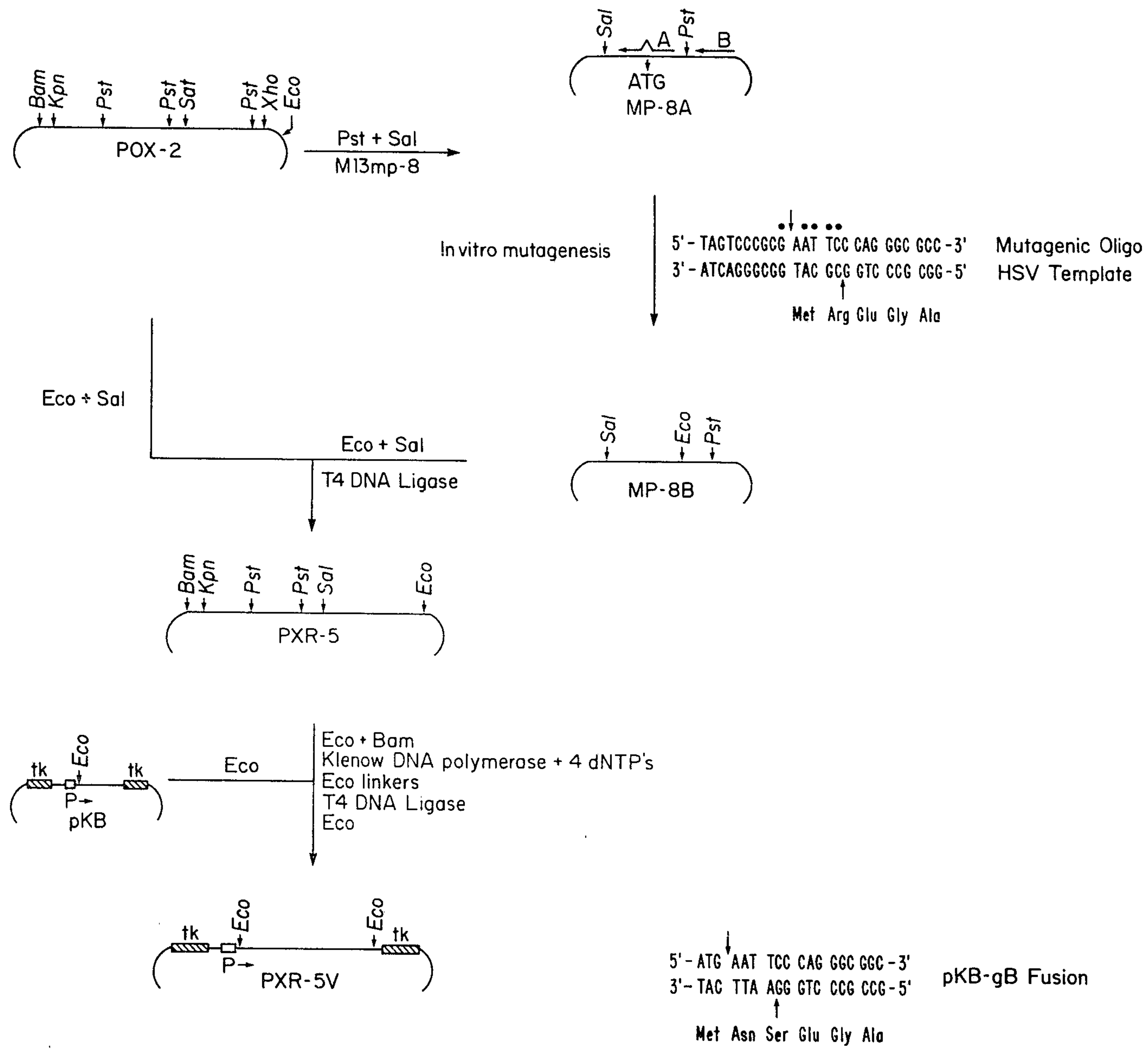

14.10.88

0297924

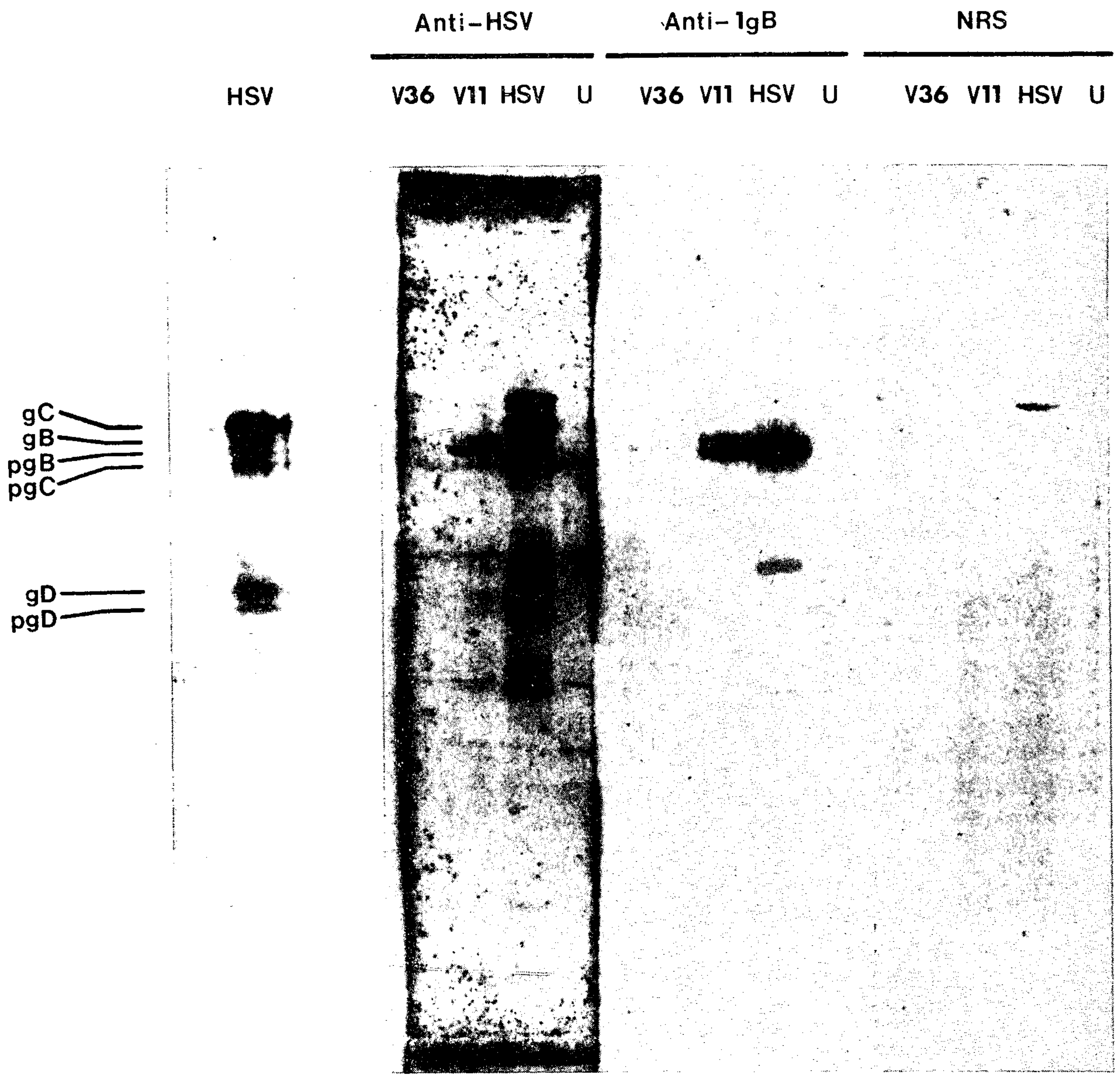


FIG. 2 A

14.10.88

0297924

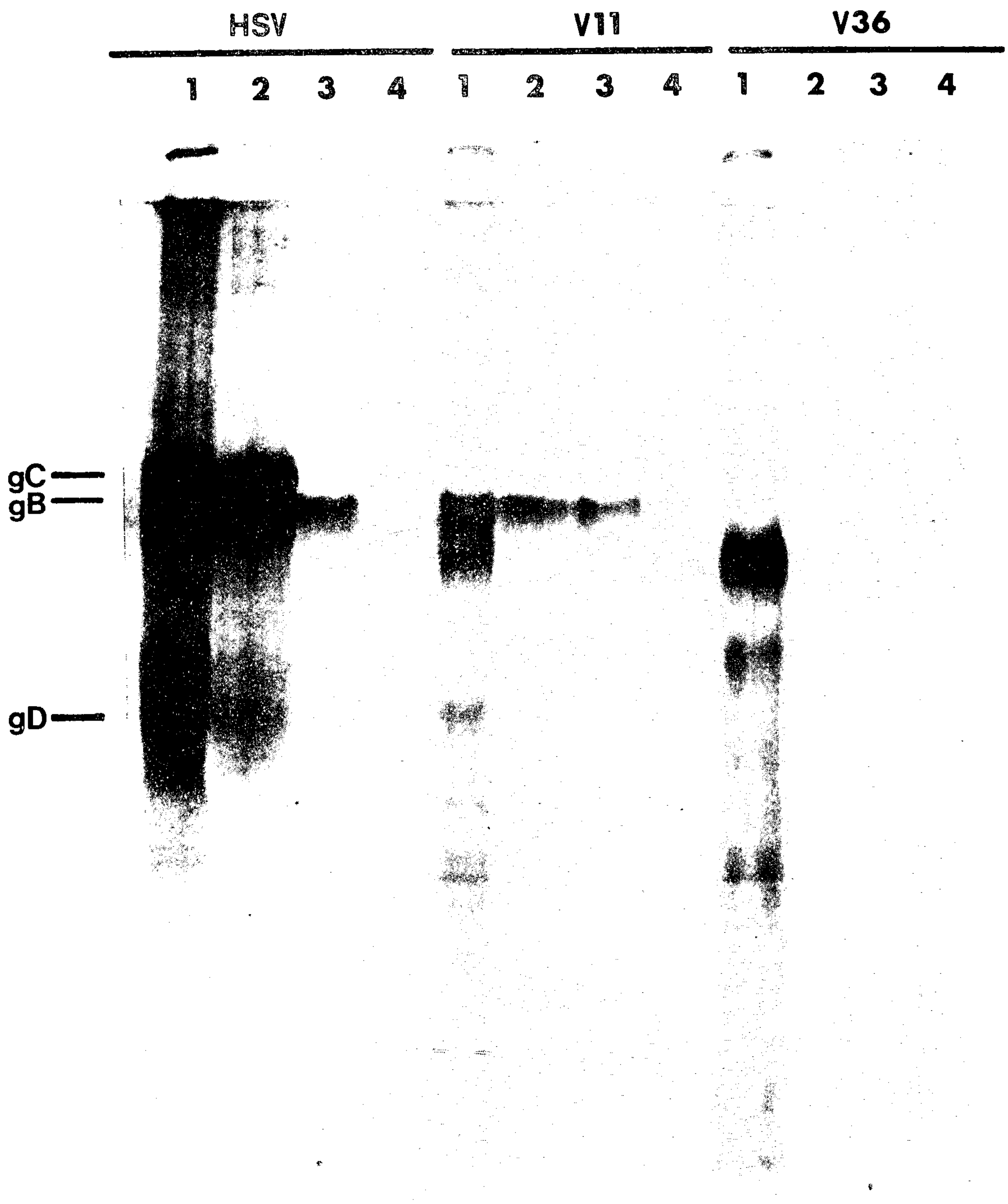


FIG. 2 B

14.10.88

0297924

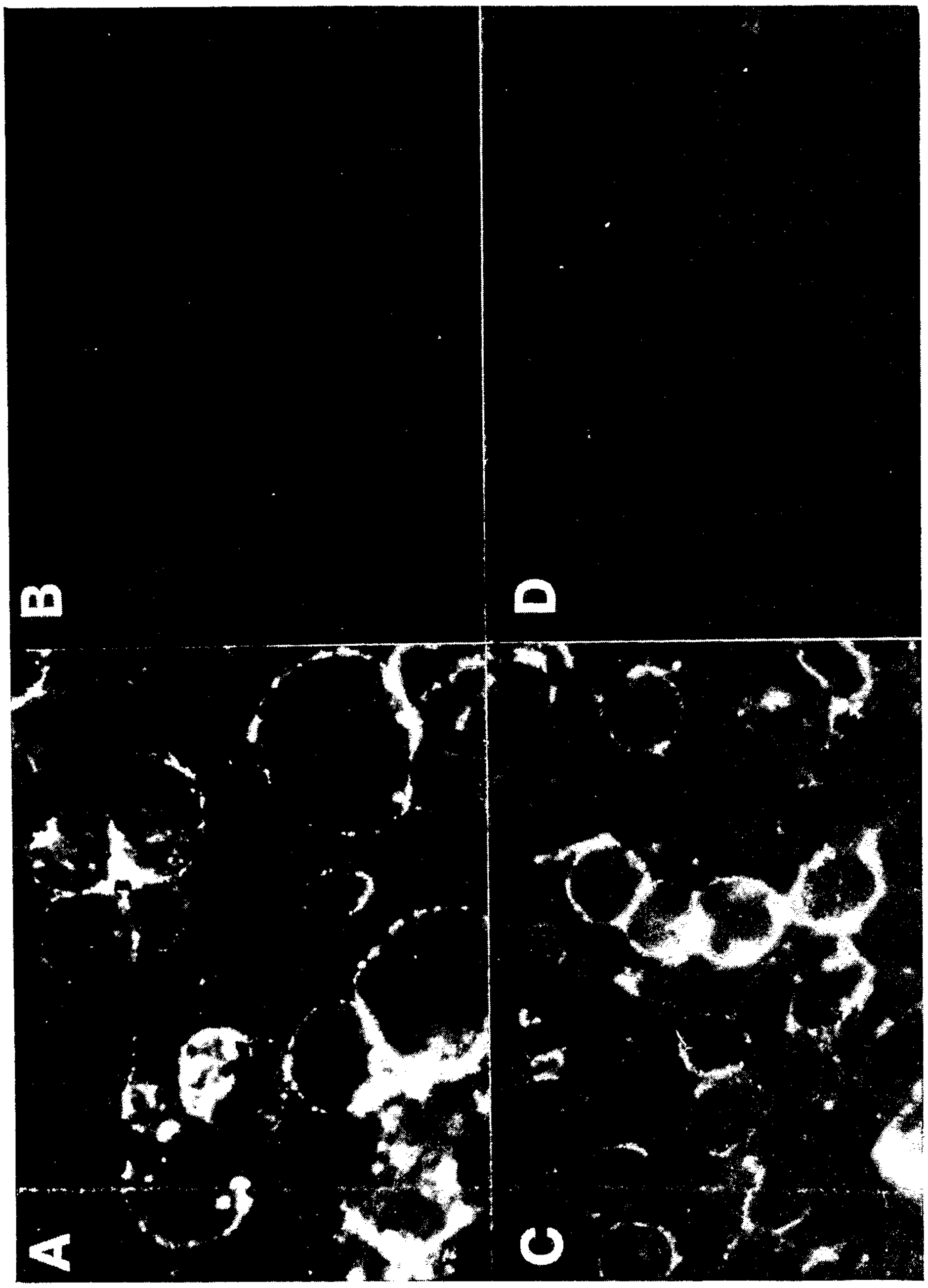


FIG. 3